# EUROPEAN PATENT APPLICATION

(11) **EP 2 901 949 A1**
(43) Date of publication of application: **05.08.2015**
(21) Application number: 14153451.1
(22) Date of filing: 31.01.2014
(51) Int. Cl.: A61B 17/28, A61B 17/3201

(54) **Injection moulded pliers**

(71) Applicant: Imperial Ventures B.V., 4624 VD Bergen op Zoom (NL)
(72) Inventor: Keijzer, Hendrikus Johannes, 4624 VD Bergen op Zoom (NL)
(74) Representative: 't Jong, Bastiaan Jacob

(57) **Abstract**

The invention relates to injection moulded pliers comprising two crossed, hingedly arranged first and second beams each having a jaw end, a handle end and a hinge section arranged between both ends, wherein:
- the hinge section of the first beam comprises two parallel spaced apart walls bridging the jaw end and the handle end, and an axle extending between the two walls and wherein:
- the hinge section of the second beam comprises a V-shaped slot terminating in a cylindrical cavity accommodating the axle of the first hinge section.

## Description

The invention relates to injection moulded pliers, in particular for use in medical application. In this field pliers are known as for example needle holders, hemostats, clamps or scissors.

Typically such tools are made of a metal, such as stainless steel. The advantage of this material is that it is durable and can easily be disinfected for reuse. However, a plurality of tools are disposed after single use, as collecting the used tools, disinfecting them and repackaging in a sterile packaging costs more than buying new ones.

However, the costs of waste disposal increase year after year. In particular grey waste, which contains anything, which cannot be separately collected, has relative high costs for disposal.

As the tools are used as disposables, i.e. for single use, it would be desired to reduce the manufacturing costs to compensate for the increasing waste disposal costs. An obvious step would be to manufacture the known pliers from a low cost material, such as a plastic. However, the design of the current tools does not enable manufacturing from a plastic, as this would cause a reduction in the handling quality of the tool.

For example, when a known needle holder would be made out of a plastic, than it will no longer be able to reliably hold a needle between its jaws. This is mainly caused by the flexibility of the plastic material, which is in particular clear in the hinge of the needle holder. Due to the increased flexibility, there will be play in the hinge, such that no sufficient force can be exerted via the handles on to the jaws.

In the medical field it is known to manufacture some tools by injection moulding, but those tools, such as a hose clamp, are less critical in relation to play or flexibility of the material.

It is now an object of the invention to provide injection moulded pliers, in which the above mentioned disadvantages are reduced or even removed.

This object is achieved with injection moulded pliers comprising two crossed, hingedly arranged first and second beams each having a jaw end, a handle end and a hinge section arranged between both ends, wherein:
- the hinge section of the first beam comprises two parallel spaced apart walls bridging the jaw end and the handle end, and an axle extending between the two walls and wherein:
- the hinge section of the second beam comprises a V-shaped slot terminating in a cylindrical cavity accommodating the axle of the first hinge section.

With the invention, the hinge of the injection moulded pliers is composed out of both hinge sections of the first and second beam.

The two walls of the hinge section of the first beam provide guide surfaces for guiding the second beam during the hinging rotation and increases the stability of the hinge. The V-shaped slot terminating in a cylindrical cavity ensures that the axle extending between the two walls of the first beam is provided with an accurate bearing surface. This bearing surface is provided by the cylindrical cavity. The bearing surface further enhances the stability of the hinge.

Another advantage, is that the injection moulded pliers according to the invention can be injection moulded in only two parts. By sliding the second beam with the jaw end between the two walls and with the V-shaped slot over the axle extending between the two walls, the pliers according to the invention can easily be assembled. In addition to the low costs of injection moulding, this will further reduce the manufacturing costs.

A further advantage of injection moulded pliers is that the plastic material has a warmer feel, than the metal material of prior art pliers. This contributes to a better user experience and also, in case of medical use, more comfort for the patient.

In a preferred embodiment of the injection moulded pliers according the invention the width of the passage from the V-shaped slot to the cylindrical cavity is smaller than the diameter of the axle to provide a snap connection.

Although the V-shaped slot terminating in a cylindrical cavity will provide usable pliers, it is of advantage if both beams are securely attached to each other by a snap connection. As the pliers according to the invention are injection moulded, the increased flexibility of the material can be used in an advantageous way by providing the snap connection.

In a further preferred embodiment of the injection moulded pliers according to the invention the distance between the two parallel spaced apart walls is equal to the thickness of the hinge section of the second beam, seen in axial direction of the axle.

By having the thickness of the second hinge section equal to the distance between the two parallel spaced apart walls, the only degree of freedom the two beams will have relative to each other is the rotation around the axle.

Preferably, the hinge section of the second beam has a constant thickness over at least the zone overlapping with the hinge section of the first beam, seen in axial direction of the axle.

This ensures that the second beam can be freely rotated over a full range, without any limitations.

In yet another embodiment of the injection moulded pliers according to the invention the handle ends of the first and second beam each comprise a grip shaped shell reinforced on the inside with a plurality of ribs.

The grip shaped shells on both beams reduces the amount of material to mould the pliers. As a result the material costs and the weight of the pliers is reduced.

The ribs further increase the strength of the shell, such that sufficient force can be transferred from the hand of an operator on to the pliers.

Another embodiment of the injection moulded pliers according to the invention further comprises two cooperating locking parts arranged to the handle ends of the first and second beams to lock the relative rotation of the beams.

Especially for needle holders it is required that the pliers can be locked in a clamping position, such that a surgeon does not need to constantly apply pressure and can focus on another part of the required operations.

Preferably, the two cooperating locking parts are toothed. The toothed parts easily interlock and by bending the beams out of the plane of rotation, the locking parts are easily disengaged.

An additional advantage of the invention, is that the beams are more flexible such that the locking parts can be more easily locked and unlocked. It also provides the possibility to use relatively larger teeth to ensure easier locking.

In still another embodiment of the injection moulded pliers according to the invention the jaw ends of the first and second beam each have a contact surface directed towards each other for clamping an object in between.

These the contact surfaces are preferably profiled to provide more grip when for example a needle is clamped.

The contact surfaces could also comprise cutting elements. In such an embodiment, the pliers can be used as scissors.

In yet another preferred embodiment of the injection moulded pliers according to the invention each beam is manufactured by injection moulding a biobased or biodegradable plastic.

The biobased or biodegradable plastic could be for example PPS (poly-phenylene sulfide), PBT (polybutylene terephthalate), PBS or PLA (poly-lactic acid). Such plastics are already available on the market. However, improvements to mechanical properties of some of these plastics may be required to further optimize the pliers according to the invention.

By injection moulding the pliers from a biobased or biodegradable plastic, disposed pliers could be discarded as bio-waste. Bio-waste has lower disposal costs as such waste can be decomposed by biological processes and has less impact on the environment.

These and other features of the invention will be elucidated in conjunction with the accompanying drawings.
Figure 1 shows a perspective view of an embodiment according to the invention.
Figure 2 shows in perspective view the embodiment of figure 1 in disassembled state.
Figure 3A - 3C show schematically three steps for assembling the embodiment of figure 1 and 2.

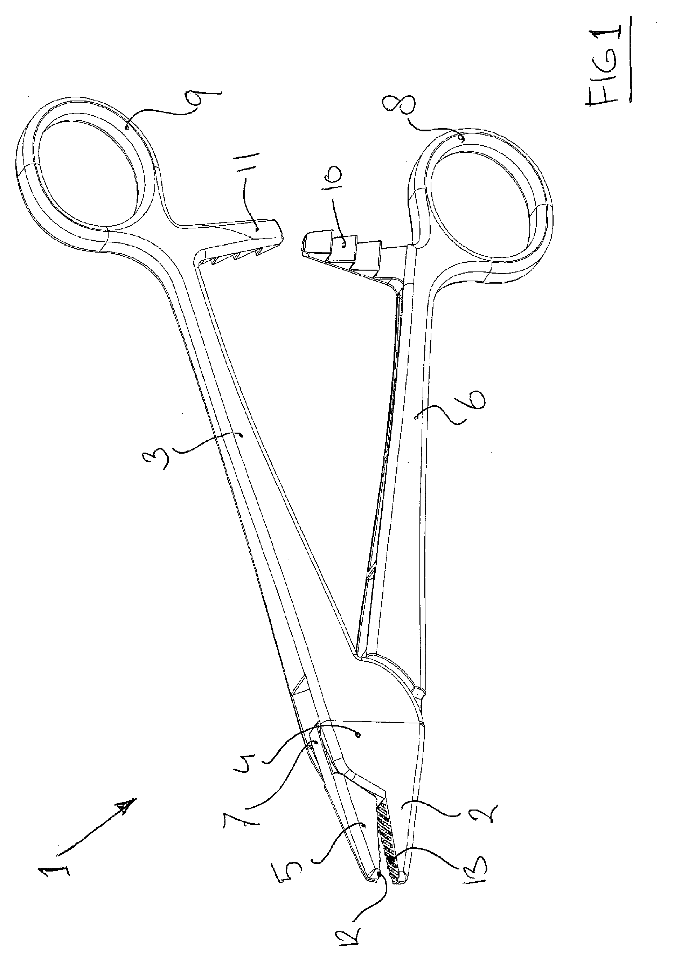

Figure 1 shows a perspective view of an embodiment of injection moulded pliers 1 according to the invention. The pliers 1 has a first beam with a jaw end 2, a handle end 3 and a hinge section 4. The second beam, which is crossed with the first beam 2, 3, 4, has also a jaw end 5, a handle end 6 and a hinge section 7.

Both handle ends 3, 6 are provided with eyes 8, 9 through which fingers of a surgeon can extend. The handle ends 3, 6 are also provided with toothed locking parts 10, 11 for locking the pliers 1 in clamped position.

The jaw ends 2, 5 of the pliers 1 each have a contact surface 12, 13 directed towards each other for clamping an object in between. The contact surfaces 12, 13 are profiled to enhance the grip, for example when a needle is clamped between the contact surfaces 12, 13.

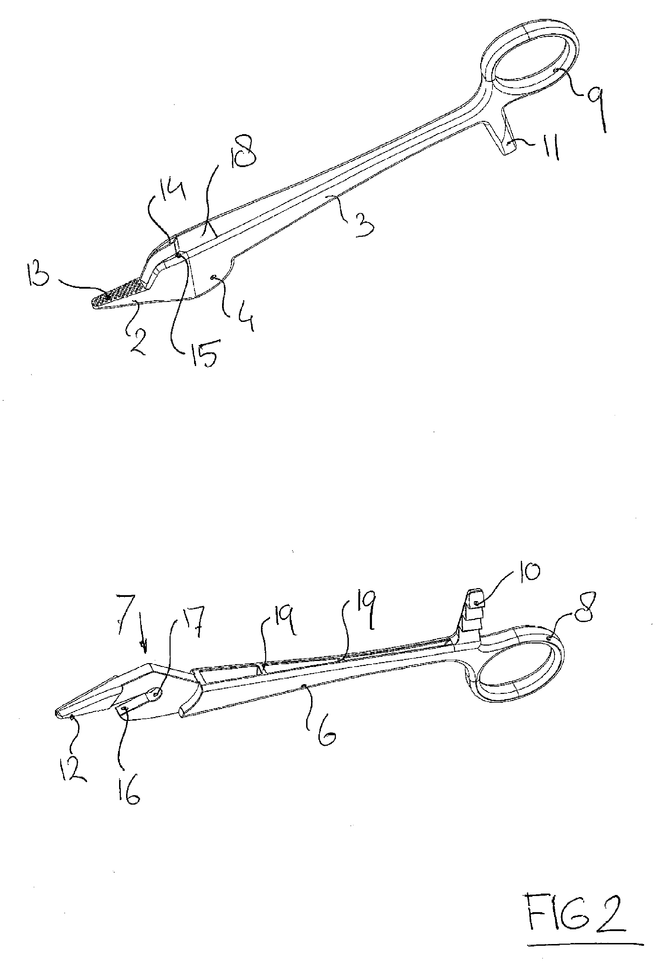

Figure 2 shows in perspective view the pliers 1 of figure 1 in disassembled state.

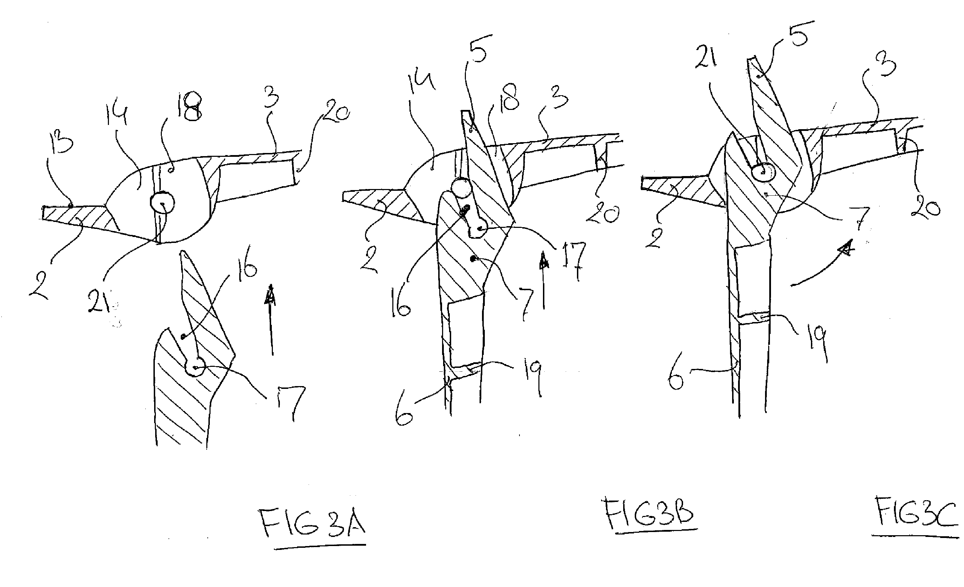

The hinge section 4 of the first beam 2, 3, 4 has two parallel walls 14, 15. An axle 21 extends between these two parallel walls 14, 15 (see figure 3A).

The hinge section of the second beam 5, 6, 7 has a V-shaped slot 16, which terminates in a cylindrical cavity 17. It is clear from figure 2 that the thickness of the second hinge section is constant, which ensures that the second hinge section 7 can be guided between the walls 14, 15.

Behind the walls 14, 15 towards the handle end 3 a wider space 18 is available such that the jaw end 12 can pass during assembling the pliers 1 (see also figure 3).

Especially clear for the second beam 5, 6, 7 is that the handle end 6 has a grip shaped shell which is reinforced on the inside with a plurality of ribs 19. Also the first beam 2, 3, 4 has a handle end 3 having a grip shaped shell reinforced with ribs 20 (see figure 3).

Figure 3A - 3C show schematically three steps for assembling the pliers 1 of figure 1 and 2. The second beam 5, 6, 7 is vertically inserted with the jaw end 5 into the space 18, such that axle 21 slides into the V-shaped slot 16. At the same time, the hinge section 7 is shifted in a sliding fit between the parallel walls 14, 15 of the first hinge section 4.

The second beam 5 is pushed further into the space 18 and between the parallel walls 14, 15, such that the axle 21 arrives at the passage between the V-shaped slot 16 and the cylindrical cavity 17. Due to the flexibility of the moulding material (typically a plastic), the axle 21 will snap into the cylindrical cavity 17 (see figure 3C). The second beam 5, 6, 7 can then be rotated around the axle 21 relative to the first beam 2, 3, 4 and the pliers 1 can be used.

## Claims

1. Injection moulded pliers comprising two crossed, hingedly arranged first and second beams each having a jaw end, a handle end and a hinge section arranged between both ends, wherein:
- the hinge section of the first beam comprises two parallel spaced apart walls bridging the jaw end and the handle end, and an axle extending between the two walls and wherein:
- the hinge section of the second beam comprises a V-shaped slot terminating in a cylindrical cavity accommodating the axle of the first hinge section.

2. Injection moulded pliers according to claim 1, wherein the width of the passage from the V-shaped slot to the cylindrical cavity is smaller than the diameter of the axle to provide a snap connection.

3. Injection moulded pliers according to claim 1 or 2, wherein the distance between the two parallel spaced apart walls is equal to the thickness of the hinge section of the second beam, seen in axial direction of the axle.

4. Injection moulded pliers according to claim 3, wherein the hinge section of the second beam has a constant thickness over at least the zone overlapping with the hinge section of the first beam, seen in axial direction of the axle.

5. Injection moulded pliers according to any of the preceding claims, wherein the handle ends of the first and second beam each comprise a grip shaped shell reinforced on the inside with a plurality of ribs.

6. Injection moulded pliers according to any of the preceding claims, further comprising two cooperating locking parts arranged to the handle ends of the first and second beams to lock the relative rotation of the beams.

7. Injection moulded pliers according to claim 6, wherein the two cooperating locking parts are toothed.

8. Injection moulded pliers according to any of the preceding claims, wherein the jaw ends of the first and second beam each have a contact surface directed towards each other for clamping an object in between.

9. Injection moulded pliers according to claim 8, wherein the contact surfaces are profiled.

10. Injection moulded pliers according to claim 8, wherein the contact surfaces comprise cutting elements.

11. Injection moulded pliers according to any of the preceding claims, wherein each beam is manufactured by injection moulding a biobased or biodegradable plastic.
